# EUROPÄISCHE PATENTSCHRIFT

(11) **EP 1 933 796 B1**
(45) Veröffentlichungstag und Bekanntmachung des Hinweises auf die Patenterteilung: **28.07.2010**
(21) Anmeldenummer: 06753961.9
(22) Anmeldetag: 30.05.2006
(51) Int. Cl.: A61F 13/49

(54) **VERFAHREN ZUM HERSTELLEN EINER VIELZAHL VON EINEN WINDELHAUPTTEIL UND DARAN ANGEFÜGTE VORDERE UND HINTERE WINDELSEITENTEILE AUFWEISENDEN INKONTINENZWEGWERFWINDELN**
METHOD OF PRODUCING A MULTIPLICTY OF INCONTINENCE PADS HAVING A MAIN PART AND FRONT AND REAR SIDE PARTS ATTACHED THERETO
PROCEDE DE PRODUCTION D'UNE PLURALITE DE COUCHES JETABLES D'INCONTINENCE COMPORTANT UN ELEMENT PRINCIPAL ET DES ELEMENTS LATERAUX AVANT ET ARRIERE ASSEMBLES A L'ELEMENT PRINCIPAL

(30) Priorität: 12.10.2005 DE 102005048868
(43) Veröffentlichungstag der Anmeldung: 25.06.2008
(73) Patentinhaber: Paul Hartmann AG, 89504 Heidenheim (DE)
(72) Erfinder: HORNUNG, Fridmann, 73466 Lauchheim (DE); KESSELMEIER, Rüdiger, 89542 Herbrechtingen (DE)
(74) Vertreter: Friz, Oliver
(86) Internationale Anmeldenummer: PCT/EP2006/005125
(87) Internationale Veröffentlichungsnummer: WO 2007/042084

(56) Entgegenhaltungen:
- EP-A- 0 923 920

## Beschreibung

Die Erfindung betrifft ein Verfahren zum Herstellen einer Vielzahl von einen Windelhauptteil und daran angefügte vordere und hintere Windelseitenteile aufweisenden Inkontinenzwegwerfwindeln.

Bei Inkontinenzwegwerfwindeln, also bei Hygieneartikeln, die üblicherweise bei Erwachsenen, insbesondere im fortgeschrittenen Alter, eingesetzt werden, sind die angefügten Windelseitenteile typischerweise ausladend. Sie werden zur korrekten Positionierung des Hygieneartikels am Körper verwendet und können auch elastisch oder elastifiziert ausgebildet sein.

Wegwerfwindeln mit vorderen im wesentlichen undehnbaren und hinteren dehnbaren beidseits angefügten Windelseitenteilen sind beispielsweise aus EP-A-0 923 920 bekannt.

WO-A-2005/110314 offenbart ein Verfahren für die endlose Rücken-an-Vorderteil-Fertigung von Wegwerfwindeln, wobei die vorderen und hinteren Windelseitenteile aus derselben Materialbahn unter Reduzierung des Schnittabfalls hergestellt werden.

Der vorliegenden Erfindung liegt die Aufgabe zugrunde, ein kostengünstig und prozesstechnisch vorteilhaft realisierbares Verfahren zum Herstellen derartiger Inkontinenzwegwerfwindeln zu schaffen.

Diese Aufgabe wird erfindungsgemäß gelöst durch ein Verfahren mit den folgenden Verfahrensschritten:
- Zuführen und Fördern einer endlosen Windelhauptteilbahn in einer ersten Längsrichtung zu einer Applikationsstation,
- Zuführen einer endlosen Windelseitenteilbahn mit einem ersten endlosen Trägermaterial, in der gegenüber dem ersten Trägermaterial dehnbare Bereiche in der Längsrichtung der Windelseitenteilbahn alternierend, das heißt mit einem Abstand voneinander, vorgesehen werden,
- Abtrennen von Längsabschnitten der Windelseitehteilbahn-und Zuführen der Längsabschnitte zu der Applikationsstation und Positionieren an der Windelhauptteilbahn,
- Unlösbares Fixieren der Längsabschnitte der Windelseitenteilbahn an ersten Bereichen der Windelhauptteilbahn, wobei die ersten Bereiche jeweils einen vorderen Hüftbereich und einen hinteren Hüftbereich zweier in Längsrichtung aufeinanderfolgender und aneinander angrenzender Windelhauptteile umfassen,
- Vereinzeln der Inkontinenzwegwerfwindeln durch Trennen der Windelhauptteilbahn quer zur Längsrichtung, wobei das Trennen durch die Längsabschnitte hindurchgeführt wird, derart dass ein erster Teilabschnitt eines jeweiligen Längsabschnitts ein vorderes Windelseitenteil einer ersten Inkontinenzwegwerfwindel bildet und ein zweiter Teilabschnitt des jeweiligen Längsabschnitts ein hinteres Windelseitenteil einer unmittelbar angrenzenden zweiten Inkontinenzwegwerfwindel bildet,
- wobei das hintere Windelseitenteil eine höhere Dehnbarkeit aufweist als das vordere Windelseitenteil.

Unter Dehnung wird das Verhältnis zwischen einer Längenzunahme eines Windelseitenteils infolge einer Krafteinwirkung und der ursprünglichen Länge verstanden. In Gebrauch derartiger Inkontinenzwegwerfwindeln wirken auf die Windelseitenteile insbesondere Kräfte in Umfangs- also Windelquerrichtung. Mit der Eigenschaft Dehnbarkeit ist somit das Ausmaß der Dehnung des Windelseitenteils bei Einwirken einer Kraft in Windelquerrichtung gemeint. Das heißt je höher das Ausmaß der Dehnung desto höher ist die Dehnbarkeit. Erfindungsgemäß weist ein hinteres Windelseitenteil also bei in Gebrauch der Windel üblicher Krafteinwirkung eine größere Dehnbarkeit auf als ein vorderes Windelseitenteil. Insbesondere weist nach einer noch zu beschreibenden Prüfmethode ein hinteres Windelseitenteil bei einer Krafteinwirkung von 45 N eine höhere Dehnung auf als ein vorderes Windelseitenteil. Vorzugsweise weist ein hinteres Windelseitenteil bei einer Krafteinwirkung von 45 N eine Dehnung von mindestens 20 %, insbesondere mindestens 25% und weiter insbesondere mindestens 30 % auf. Ein vorderes Windelseitenteil hingegen weist bei einer Krafteinwirkung von 45 N lediglich eine Dehnung von vorzugsweise höchstens 15%, insbesondere höchstens 10% und weiter insbesondere von höchstens 8% auf.

Vorzugsweise ist zumindest ein hinteres Windelseitenteil zumindest in Querrichtung elastisch dehnbar. Die Dehnbarkeit des Windelseitenteils wird als elastisch bezeichnet, wenn bei kurzzeitiger Einwirkung einer Kraft eine Dehnung von mindestens 40% möglich ist und bei wegnahme dieser Kraft eine Dehnung (bleibende Dehnung) von höchstens 20 % verbleibt.

In einer vorteilhaften Weiterbildung der Erfindung beträgt die elastische Dehnbarkeit eines hinteren Windelseitenteils in Querrichtung mindestens 40%, insbesondere mindestens 50%. Nach einem weiteren Erfindungsgedanken beträgt das absolute Ausmaß der elastischen Dehnung eines hinteren Windelseitenteils mindestens 3 cm, insbesondere mindestens 5 cm und weiter insbesondere mindestens 7 cm.

Nach dem erfindungsgemäßen Verfahren wird also eine Windelhauptteilbahn endlos gefördert, und an diese Windelhauptteilbahn werden in der beanspruchten Weise Windelseitenteile in der Herstellungsmaschine angefügt. Die Windelseitenteile stammen ihrerseits von einer endlos zugeführten Windelseitenteilbahn, von der Längsabschnitte abgetrennt und dann auf die endlos geförderte Windelhauptteilbahn appliziert und an dieser fixiert werden. Erfindungsgemäß wird zum Vereinzeln der Inkontinenzwegwerfwindeln ein Trennschnitt durch einen jeweiligen Längsabschnitt hindurchgeführt, so dass der eine Teilabschnitt dieses Längsabschnitts zu dem einen Inkontinenzartikel und der andere Teilabschnitt zum angrenzenden Inkontinenzartikel gehört. In vorteilhafter Weise werden die betreffenden Bereiche der Windelseitenteilbahn im Hinblick auf ihre Dehnbarkeit, wie bereits erläutert, so ausgebildet und die erwähnten Längsabschnitte von der Windelseitenteilbahn so abgetrennt und der Trennschnitt beim Vereinzeln der Inkontinenzwegwerfartikel so geführt, dass ein hinteres Windelseitenteil, also ein Windelseitenteil, welches an den hinteren Hüftbereich oder Rückenbereich des Windelhauptteils angefügt ist, eine höhere Dehnbarkeit aufweist als ein vorderes Windelseitenteil, welches an einen vorderen Hüftbereich oder Bauchbereich des Windelhauptteils angefügt ist. Es wurde nämlich festgestellt, dass durch eine solche differenzierte Dehnbarkeit eine bessere Passform des Inkontinenzartikels erreicht werden kann, oder anders ausgedrückt, dass keine Notwendigkeit für die Schaffung von einander entsprechenden Dehnbarkeiten im vorderen und hinteren Windelseitenteil besteht. Da die Bereitstellung von Dehnbarkeitseigenschaften, insbesondere elastischer Dehnbarkeitseigenschaften, stets mit Kosten verbunden ist, erweist sich die Realisierbarkeit einer differenzierten Dehnbarkeit bei vorderen und hinteren Windelseitenteilen schon aus diesem Grund als vorteilhaft. Dies erfordert jedoch die Bereitstellung einer variierten Dehnbarkeit bei der Windelseitenteilbahn. Dies wird vor dem Applizieren der Längsabschnitte der Windelseitenteilbahn auf die Windelhauptteilbahn, realisiert, was an späterer Stelle noch im Einzelnen in Verbindung mit den Ausführungsbeispielen beschrieben werden wird.

Der Windelhauptteil bzw. die Windelhauptteilbahn können prinzipiell in der Längsrichtung kontinuierlich insbesondere aus Vlies- oder Folienmaterial oder aus einem Vlies/FolienVerbund hergestellt werden. Es erweist sich aber auch als vorteilhaft, wenn ein jeweiliger Windelhauptteil einen Flüssigkeiten speichernden, vorzugsweise superabsorbierende Materialien umfassenden Absorptionskern aufweist, der insbesondere als vorgefertigte Einheit auf eine Trägerbahn der Windelhauptteilbahn aufgebracht werden kann.
Beispielsweise könnte diese Trägerbahn eine Folienschicht umfassen oder auch eine Verbundfolie mit einer dünnen Vliesstoffauflage auf der späteren Außenseite, wobei dann auf diese Trägerbahn in Längsrichtung aufeinanderfolgend und in einem Abstand zueinander Absorptionskerne aufgebracht und vorzugsweise gegen die Trägerbahn fixiert werden. Dies kann wie erwähnt ein vorkonfektionierter Absorptionskern sein, häufig auch als Saugkörper bezeichnet, oder der Absorptionskern kann durch Ablage von absorbierendem Fasermaterial vorzugsweise mit superabsorbierenden Polymermarterialien konstruktiv gebildet sein.

Des Weiteren erweist es sich als vorteilhaft, dass in der ersten Längsrichtung erstreckte erste elastische Elemente an die Windelhauptteilbahn angefügt werden, und zwar auf beiden Seiten. Diese elastischen Elemente können auch einer gewissen Konturierung entlang der Beinöffnungen folgend vorgesehen werden. Sie können aber auch exakt in Längsrichtung verlaufen.

Des Weiteren können in der ersten Längsrichtung erstreckte zweite elastische Elemente, insbesondere in Form von sogenannten und an sich zum Beispiel aus EP0263720A1 bekannten aufstehenden Cuff-Elementen, an die Windelhauptteilbahn angefügt werden. Diese vorzugsweise aufstehenden zweiten elastischen Elemente flankieren gewissermaßen ein Zentrum des Windelhauptteils oder des Saugkörpers; sie können im Bereich der Saugkörperränder, innerhalb der Saugkörperränder oder außerhalb der Saugkörperränder vorgesehen werden. Sie bilden einen Seitenauslaufschutz des Inkontinenzartikels.

Nach einer vorteilhaften Variante des erfindungsgemäßen Verfahrens erfolgt das Zuführen der Längsabschnitte zu der Applikationsstation mit einer ernsten Geschwindigkeit v1 und das Zuführen der endlosen Windelhauptteilbahn zu der Applikationsstation mit einer zweiten Geschwindigkeit v2, wobei die erste Geschwindigkeit v1 geringer ist als die zweite Geschwindigkeit v2. Solchenfalls werden die Längsabschnitte der Windelseitenteilbahn bei verhältnismäßig geringer Geschwindigkeit v1 abgetrennt und dann vorzugsweise auf die Geschwindigkeit v2 beschleunigt, so dass die Längsabschnitte vorzugsweise bei derselben Geschwindigkeit v2 auf die Windelhauptteilbahn abgelegt werden. Diese Beschleunigung kann beispielsweise durch eine insbesondere unterdruckbeaufschlagbare Applikationswalze erfolgen, die in Transportrichtung der Windelseitenteilbahn einer Trenn- oder Schneidstation, wo die Längsabschnitte abgetrennt werden, insbesondere unmittelbar nachfolgend angeordnet ist.

Die zweite Geschwindigkeit kann nach einer bevorzugten Ausführungsform der Erfindung um mindestens 40%, insbesondere um mindestens 70%, insbesondere um mindestens 90%, insbesondere um höchstens 200% größer sein als die erste Geschwindigkeit v1.

Nach einer weiteren Ausführungsform von besonderer Bedeutung kann die Windelseitenteilbahn ein erstes Trägermaterial umfassen, welches ein regelmäßiges Muster insbesondere fensterartiger Ausnehmungen aufweist, die von elastisch dehnbarem Material bzw. elastischen Elementen wie beispielsweise elastischen Folien, oder elastischen Vliesen überfangen sind. Dadurch, dass in das Trägermaterial Ausnehmungen eingebracht werden, die von elastischem Material im weitesten Sinn überfangen werden, lässt sich eine von der Dehnbarkeit des Trägermaterials abweichende Dehnbarkeit erreichen. Wenn beispielsweise das Trägermaterial im Wesentlichen undehnbar, wenig dehnbar oder zumindest weniger dehnbar ist als das herzustellende Windelseitenteil sein soll, so weist das auf die beschriebene Weise modifizierte Trägermaterial mit Öffnungen, welche von elastischem Material überfangen sind, eine größere Dehnbarkeit als das ursprüngliche Trägermaterial auf.

Die erwähnten Ausnehmungen können in dem Trägermaterial der Windelseitenteilbahn schon vor dem Eintritt in die Herstellungsmaschine vorgesehen sein, oder sie werden in vorteilhafter Weise erst unmittelbar bei der Herstellung der erfindungsgemäßen Inkontinenzwegwerfwindeln in der Windelherstellungsmaschine ausgeschnitten oder ausgestanzt. Letzteres ist insoweit vorteilhaft, als das Trägermaterial von der Rolle ohne vorherige. Vorpositionierung endlos in die Herstellungsmaschine eingeführt werden kann, was auch einen Rollenwechsel weniger problematisch erscheinen lässt. Auch das elastische Material bzw. die elastischen Elemente können in dem Trägermaterial der Windelseitenteilbahn über die Ausnehmungen in dem Trägermaterial schon vor dem Eintritt in die Herstellungsmaschine aufgebracht und gegenüber dem Trägermaterial fixiert vorgesehen sein, oder sie werden in vorteilhafter Weise erst unmittelbar bei der Herstellung der erfindungsgemäßen Inkontinenzwegwerfwindeln innerhalb der Maschine über die Ausnehmungen in dem Trägermaterial aufgebracht und gegenüber dem Trägermaterial fixiert.

Die Ausnehmungen werden in das Trägermaterial derart eingebracht und die Längsabschnitte werden derart von der Windelseitenteilbahn abgetrennt, dass die Ausnehmungen in vorteilhafter Weise nur innerhalb der zweiten Teilabschnitte angeordnet sind. Ein jeweiliger erster Teilabschnitt ist somit in Bezug auf die Dehnbarkeitseigenschaften des ersten Trägermaterials im Wesentlichen unverändert. Der jeweilige zweite Teilabschnitt hingegen ist infolge des die Ausnehmung überfangenden elastischen Materials dehnbar. Durch das Aufbringen des elastischen Materials oder elastischer Elemente wird also eine elastische Dehnbarkeit geschaffen.

Durch das Vorsehen von Ausnehmungen in der Windelseitenteilbahn kann mit einem in der Längsrichtung endlosen kontinuierlichen Trägermaterial gearbeitet werden, in welches dann entsprechend der Lage der abzutrennenden Längsabschnitte und der Lage der Teilabschnitte dieser Längsabschnitte Ausnehmungen ausgebildet werden, die von elastischem Material überfangen sind. Es kann also bei einer in Längsrichtung durchgehenden kontinuierlichen Bahn eine differenzierte Dehnbarkeit erreicht werden.

Eine weitere Möglichkeit, eine bereichsweise Modifizierung der Dehnbarkeitseigenschaften zu erreichen, besteht darin, dass die Windelseitenteilbahn wiederum bereichsweise und alternierend vorzugsweise durch eine als "ring rolling" bekannt gewordene Technologie geschwächt wird. Diese Technologie ist beispielsweise in EP 0 573 586 B1 und EP 0 650 714 A1 beschrieben. Durch "ring rolling" wird ein an sich nicht dehnbares Material, beispielsweise ein Folienmaterial oder ein vlies/Folien-Laminat durch übermäßige Auslenkung zwischen gegeneinander kämmenden walzen überdehnt. In diesem überdehnten Zustand bringt das zuvor an sich nicht dehnbare Material einer Längung im Wesentlichen keinen Widerstand entgegen. Durch Kombination mit einem elastisch dehnbaren Element kann somit eine elastische Dehnbarkeit in dem entsprechend behandelten Bereich erreicht werden. Insofern erweist es sich als vorteilhaft, dass die elastischen Bereiche der Windelseitenteilbahn durch Aufbringen von elastischem Material auf das Trägermaterial und bereichsweises Überdehnen des Trägermaterials gebildet werden.

Das elastisch dehnbare Material, welches bei der Windelseitenteilbahn vorgesehen werden kann, könnte in der Längsrichtung alternierend, d. h. mit einem Abstand voneinander auf der Windelseitenteilbahn angeordnet sein. Es kann sich auch als vorteilhaft erweisen, wenn die elastischen Elemente der Windelseitenteilbahn von in der Längsrichtung der Windelseitenteilbahn kontinuierlich erstrecktem, insbesondere streifenförmigem elastischen Material gebildet ist, das aber in der Längsrichtung nur abschnittsweise aktiviert wird. Diese abschnittsweise Aktivierung kann, wie vorausgehend erörtert, durch eine lokale Schwächung des Trägermaterials, beispielsweise durch Vorsehen von Ausnehmungen oder durch "ring rolling" oder in einer sonstigen Weise beispielsweise auch durch Vorsehung von Perforationen realisiert werden. Obschon die Anordnung eines in Längsrichtung kontinuierlichen elastischen Materials mit entsprechenden Materialkosten verbunden ist, erweist sich dies konstruktionstechnisch in schnelllaufenden Maschinen einfacher als eine intermittierende Aufbringung von elastischem Material.

In einer weiteren vorteilhaften Weiterbildung der Erfindung werden in dem zweiten Teilabschnitt der Windelseitenteilbahn zumindest zwei vorzugsweise genau zwei dehnbare, vorzugsweise elastisch dehnbare Bereiche vorgesehen. Insbesondere erweist es sich als vorteilhaft einen ersten dehnbaren Bereich am oder in der Nähe des oberen Querrandes des zweiten Teilabschnittes und einen zweiten dehnbaren Bereich am oder in der Nähe des unteren Querrandes des zweiten Teilabschnittes vorzusehen. Solchenfalls ist das hintere Windelseitenteil genau dort mit einer höheren Dehnbarkeit ausgestattet, wo erfahrungsgemäß in Gebrauch, beispielsweise beim Schließen der Inkontinenzwegwerfwindel, die höchstens Kräfte einwirken. Einem Abreißen der Windelseitenteile vom Hauptteil kann hierdurch vorgebeugt werden.

Das elastische Material oder elastische Materialabschnitte könnten auf einer Oberseite oder Unterseite des Trägermaterials der Windelseitenteilbahn vorgesehen werden. Es erweist sich indessen als vorteilhaft, wenn das elastische Material sandwichartig zwischen einem ersten und einem zweiten Trägermaterial der Windelseitenteilbahn angeordnet ist.

Die Windelseitenteilbahn oder der erwähnte Längsabschnitt der Windelseitenteilbahn könnte vor dem Applizieren auf die Windelhauptteilbahn in der Längsrichtung in einen linken und einen rechten Teil getrennt werden, die dann an einen linken bzw. rechten Seitenrand der Windelhauptteilbahn appliziert und dort befestigt werden. Nach einer anderen Ausführungsform ist es jedoch denkbar, dass ein Längsabschnitt der Windelseitenteilbahn in einer Querrichtung der Windel einstückig durchgehend ausgebildet ist, so dass er den betreffenden vorderen oder hinteren Hüftbereich des Windelhauptteils durchgehend überfängt.

Das Vereinzeln der Inkontinenzwegwerfwindeln erfolgt schließlich nach dem Applizieren und Fixieren der Teilabschnitte der windelseitenteilbahn an der Windelhauptteilbahn. Hierfür kann ein im Wesentlichen einziger Trennschnitt durch den Längsabschnitt und die Windelhauptteilbahn hindurchgeführt werden. Es wäre auch denkbar, dass der Trennschnitt nur den Längsabschnitt erfasst, nämlich dann, wenn die Windelhauptteilbahn zuvor quer zur Längsrichtung zur Bildung von vereinzelten Windelhauptteilen getrennt worden wäre, die dann in einem gewissen Abstand voneinander weiter zur Applikationsstation gefördert werden, wo der Längsabschnitt diesen Abstand brückenartig überdeckt. Der gemeinsamen Vereinzelung, bei der der Schnitt sowohl durch den Längsabschnitt der Windelseitenteilbahn als auch durch die Windelhauptteilbahn geführt wird, wird jedoch der Vorzug gegeben.

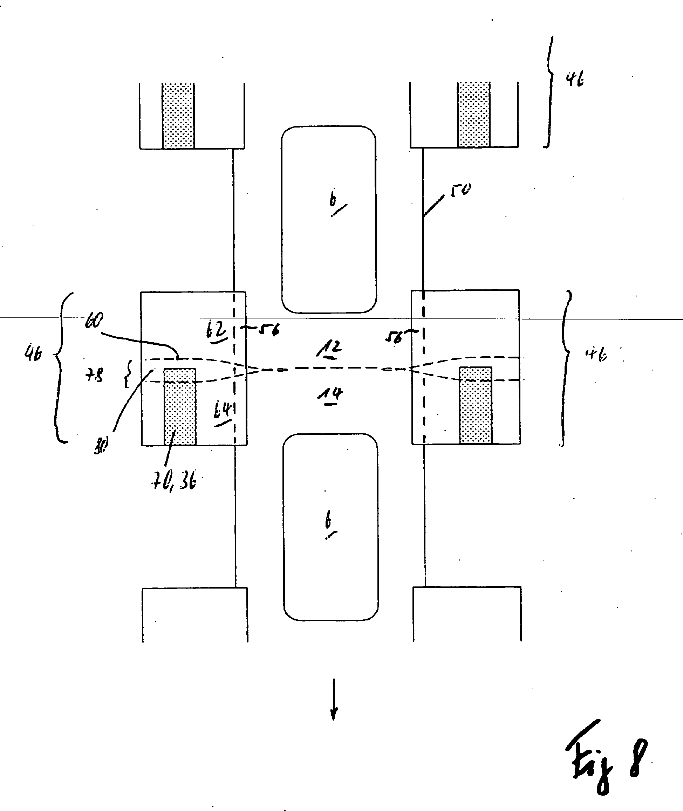

Anstelle der Ausführung eines einzigen quer zur Längsrichtung verlaufenden Trennschnitts kann es sich beim Vereinzeln der Inkontinenzwegwerfwindeln oder beim Abtrennen der Längsabschnitte als vorteilhaft erweisen, dass ein zu verwerfender Abschnitt gebildet wird. Dies wäre beispielsweise durch zwei sogenannte Konturschnitte denkbar oder durch einen beispielsweise rechteckförmig geführten (Stanz)schnitt, wobei einerseits die Bahn durchtrennt und andererseits ein rechteckförmiger zu verwerfender Abschnitt gebildet wird. Auf diese Weise geht zwar Material verloren, es kann aber eine gewisse Positionsungenauigkeit hierdurch kompensiert werden. Insbesondere kann verhindert werden, dass ein zuvor (diskontinuierlich) aufgebrachter Abschnitt aus elastischem Material nicht nur bestimmungsgemäß in dem zweiten Teilabschnitt vorgesehen ist, sondern sich in ungewollter Weise infolge des ungenauen Trennschnitts auch in den ersten Teilabschnitt erstreckt. Indem ein zu verwerfender Abschnitt einer hinreichenden Breite in der Längsrichtung von insbesondere 5 bis 20, insbesondere 5 bis 15, insbesondere 5 bis 10 mm verworfen wird, kann dies mit der erforderlichen Prozesssicherheit verhindert werden. Wenn mit von der exakt linearen Form abweichenden Konturschnitten gearbeitet wird, kann zudem eine Konturgebung, wie sie beispielsweise in Figur 8 angedeutet ist, realisiert werden.

Ungeachtet dessen erweist es sich nach einem an sich eigenständigen Erfindungsgedanken als vorteilhaft, dass die vorderen Windelseitenteile im Wesentlichen undehnbar ausgebindet werden, während die hinteren Windelseitenteile dehnbar, insbesondere elastisch dehnbar ausgebildet werden. In Weiterbildung dieses an sich eigenständigen Erfindungsgedankens, erweist es sich als vorteilhaft, wenn die Länge der hinteren Windelseitenteile, also deren Erstreckung in Windellängsrichtung mindestens 10 cm, insbesondere mindestens 15 cm, weiter insbesondere mindestens 18 cm und weiter insbesondere mindestens 22 cm beträgt. Es erweist sich weiterhin als vorteilhaft, wenn die Länge der hinteren Windelseitenteile mindestens 10 %, insbesondere mindestens 15%, weiter insbesondere mindestens 20% und weiter insbesondere mindestens 22% der Gesamtlänge der Inkontinenzwegwerfwindel beträgt. Vorteilhafterweise beträgt die Gesamtlänge der Inkontinenzwegwerfwindel 50-120 cm, insbesondere 60-110 cm und weiter insbesondere 70-110 cm. Weiterhin erweist es sich als vorteilhaft, wenn die vorderen Windelseitenteile eine geringere, insbesondere eine um mindestens 5%, weiter insbesondere eine um mindestens 10%, weiter insbesondere eine um mindestens 15% und weiter insbesondere eine um höchstens 50% geringere Längserstreckung aufweisen als die hinteren Windelseitenteile. In Weiterbildung der Erfindung erweist es sich als vorteilhaft, wenn die Breite der Windelseitenteile, also die Erstreckung der Windelseitenteile über den Seitenrand des Windelhauptteils hinaus 10-40 cm, insbesondere 12-30 cm, weiter insbesondere 13-25 cm beträgt. Vorzugsweise weist das vordere Windelseitenteil die gleiche Breite auf wie das hintere Windelseitenteil.

Die an den Windelhauptteil angefügten Windelseitenteile sind vorzugsweise zumindest bereichsweise aus einem Vliesstoff gebildet, insbesondere und vorzugsweise können Spunbond-Materialien (S) oder Spunbond-Meltblown-Materialien (SM), oder beidseitig mit Spunbond-Materialien versehene Meltblown-Schichten (SMS) oder auch kardierte Vliesmaterialien Verwendung finden. Auch Vliesstofflaminate, also insbesondere zweilagige, dreilagige oder mehrlagige Kombinationen der vorgenannten Vliesstoffe, können Verwendung finden. Die Verbindung der einzelnen Lagen kann durch an sich übliche und bekannte Verfahren, beispielsweise durch thermische Fügeverfahren (Verschweißung, insbesondere Laserverschweißung, hotmelt, air-through) oder durch Ultraschallschweißverfahren erfolgen; auch die kalte Verpressung, Vernadelung, Vernähung oder Verklebung von Vliesstoffmaterialien ist denkbar. Auch die Verbindung mit textilen Geweben, Gewirken oder Gestricken, also mit eine textile Bindung im weitesten Sinne aufweisenden Materialien sowie mit Folien und Schaumstoffen ist denkbar. Vorzugsweise werden die seitlich an den Windelhauptteil angefügten Windelseitenteile zumindest abschnittsweise atmungsaktiv ausgebildet, wobei insbesondere eine Porosität als vorteilhaft angesehen wird, die sowohl einen Luftaustausch als auch eine Durchlässigkeit für Feuchtigkeit in Form von Wasserdampf gestattet. Das Windelseitenteilmaterial hat in vorteilhafter Weise ein Flächengewicht von 10 bis 150 g/m², insbesondere 20 -100 g/m², weiter insbesondere 25 - 50 g/m².

Es erweist sich des Weiteren als vorteilhaft, dass die Windelseitenteile um mindestens eine in der ersten Längsrichtung erstreckte Faltlinie auf sich selbst und/oder auf den Windelhauptteil eingefaltet werden. Es erweist sich in Weiterbildung dieses Gedankens als vorteilhaft, wenn die Windelseitenteile wie dies in DE202004006951.2 offenbart ist in der gefalteten Konfiguration an Fügestellen oder Fügebereichen lösbar fixiert werden, insbesondere durch Ultraschweißpunkte. Auf den Offenbarungsgehalt der DE202004006951.2 wird hiermit ausdrücklich Bezug genommen. Hierdurch kann das betreffende Windelseitenteil innerhalb der Herstellungsmachine in einer prozesstechnisch beherrschbaren stabilen Konfiguration gehalten werden; ein Aufflattern kann so sicher verhindert werden.

Die Faltung und gegebenenfalls die lösbare Fixierung der Faltung der Windelseitenteile erfolgt vorzugsweise vor dem Abtrennen der Längsabschnitte. Es erfolgt somit vorzugsweise eine Faltung der noch endlosen Windelseitenteilbahn. Dies hat den Vorteil, der kompakteren Auslegung der nachfolgenden Applikationsstation.

Es erweist sich in Weiterbildung der Erfindung als besonders vorteilhaft, wenn an einem in Querrichtung das freie Ende des Windelseitenteils bildenden Teilbereich eines jeweiligen so gefalteten Windelseitenteils ein Anfassbereich zum Entfalten des Windelseitenteils vorgesehen ist. Dieser Anfassbereich kann im einfachsten Fall von einem Längsseitenrandabschnitt des erwähnten Teilbereiches gebildet sein, der mit den Fingern eines Benutzers ergreifbar ist. Es wäre auch denkbar, dass an dem betreffenden Teilbereich ein separates manuell ergreifbares Anfasselement vorgesehen ist, was jedoch einen zusätzlichen herstellungstechnischen Aufwand bedeuten würde.

In diesem Zusammenhang erweist es sich nach einer besonders vorteilhaften und bevorzugten Weiterbildung des erfindungsgemäßen Inkontinenzartikels als vorteilhaft, dass die lösbare Fixierung an sämtlichen Fügestellen oder Fügebereichen beim Entfalten durch einmaliges Ziehen an einem Anfassbereich der jeweiligen Windelseitenteile auftrennbar ist. Hierdurch wird die Handhabung weiter vereinfacht und der Inkontinenzartikel wird hierdurch noch bedienungsfreundlicher, gerade was die Anwendung bei stark pflegebedürftigen Personen betrifft.

Die vorerwähnte durch einmaliges Ziehen an einem Anfassbereich, also durch eine einzige Zugbewegung, erreichbare vollständige Entfaltung der gefalteten Windelseitenteile bedeutet, dass der Benutzer nicht mehrfach ruckartig an einem jeweiligen Windelseitenteil ziehen oder gar zerren muss, bis sämtliche Fügestellen zwischen Teilbereichen des Windelseitenteils und gegebenenfalls auch zum Windelhauptteil des Inkontinenzartikels gelöst werden.

Im einfachsten Fall ist ein jeweiliges Windelseitenteil um eine Falzlinie auf sich selbst gefaltet, so dass zwei Teilbereiche aufeinander liegen bzw. gegeneinander anliegen. Vorzugsweise ist das Windelseitenteil aber um wenigstens zwei Falzlinien auf sich selbst gefaltet, so dass eine im Schnitt Z-förmige Konfiguration entsteht. Nach einer weiteren bevorzugten Ausführungsform sind die Windelseitenteile um drei Falzlinien auf sich selbst gefaltet. Nach einer weiteren bevorzugten Ausführungsform sind die Windelseitenteile um vier Falzlinien auf sich selbst gefaltet.

Nach einer weiteren bevorzugten Ausführungsform des erfindungsgemäßen Inkontinenzartikels sind die jeweiligen Anfassbereiche vor der Entfaltung der Windelseitenteile in Querrichtung nach außen gewandt, also voneinander und von einer Längsmittelachse des auf einer ebenen Unterlage ausgebreiteten Windelhauptteils voneinander weggewandt, so dass sie auf bequeme Weise mit der linken Hand eines Benutzers von links und mit der rechten Hand von rechts ergreifbar sind.

Die lösbare Fixierung der aufeinander gefalteten Teilbereiche der Windelseitenteile aneinander und möglicherweise auch mit dem Windelhauptteil ist vorzugsweise durch mehrere im Wesentlichen punktförmige Fügestellen ausgebildet. Eine punktförmige Fügestelle der vorstehend erwähnten Art bedeutet, dass die Fügestelle eine Fläche (in Projektion auf die X-Y-Ebene des Windelhauptteils) von weniger als 5 mm², insbesondere von weniger als 2 mm² und weiter insbesondere von weniger als 1 mm² aufweist. Die Fügestellen müssen nicht streng punkt- oder kreisförmig sein. Denkbar und vorteilhaft sind auch von der Punkt - oder Kreisform abweichende Formen wie Dreieck-, Viereck-, Vieleck-, oder Ovalformen. Vorzugsweise ist die lösbare Fixierung der aufeinander gefalteten Teilbereiche der Windelseitenteile aneinander durch thermisch oder durch Ultraschall erzeugte, vorzugsweise punktförmige Fügestellen ausgebildet.

Es wurde erfindungsgemäß erkannt, dass die Anzahl, die Verteilung oder der Flächenanteil der Fügestellen oder die Haftkraft der lösbar aneinander gefügten Teilbereiche so gewählt werden kann, dass die lösbare Fixierung an sämtlichen Fügestellen oder Fügebereichen beim Entfalten durch einmaliges Ziehen an dem jeweiligen Anfassbereich der Windelseitenteile auftrennbar ist. Dies kann in vorteilhafter Weise dadurch unterstützt werden, dass die Anzahl oder der Flächenanteil der Fügestellen oder die Haftkraft der lösbar aneinander gefügten Teilbereiche mit der Entfernung von dem Anfassbereich des Windelseitenteils abnimmt. Es wurde erfindungsgemäß erkannt, dass je weiter,ein Bereich der aufeinander gefalteten Teilbereiche der Windelseitenteile von dem Anfassbereich entfernt ist, desto geringer die Stärke der Fixierung der Teilbereiche aneinander sein sollte, um eine Lösung sämtlicher Fügestellen oder - bereiche durch einmaliges Ziehen an dem jeweiligen Anfassbereich der Windelseitenteile, also durch eine einmalige Entfaltungsbewegung, zu erreichen. Es wurde demzufolge auch erkannt, dass in der Nähe des Anfassbereichs die lösbare Fixierung der aufeinander gefalteten Teilbereiche problemlos den Anforderungen entsprechend stark ausgebildet werden kann. So kann ein sicherer Transport der vorzugsweise schon vor oder in der schnelllaufenden Windelherstellungsmaschine gefalteten Flachmaterialbahnen gewährleistet werden, ohne dass vom Windelhauptteil des Inkontinenzartikels seitlich vorstehende Windelseitenteile flattern oder aufeinander gefaltete Teilbereiche innerhalb der Faltung verschoben werden. Es ergibt sich auch später bei der Faltung des gesamten Produkts ein sauberes Erscheinungsbild.

In Weiterbildung der Erfindung erweist es sich als vorteilhaft, wenn die windelseitenteile vor Ingebrauchnahme des zusammengefalteten Artikels nach innen um eine in Längsrichtung verlaufende Einschlagachse auf die körperzugewandte Seite des Windelhauptteils eingeschlagen sind um eine übereinander eingeschlagene Anordnung zu bilden, derart, dass ein erstes hinteres Windelseitenteil - beispielsweise das rechte - zumindest bereichsweise unterhalb des zweiten - beispielsweise des linken - hinteren Windelseitenteils zu liegen kommt. Hierbei kann ein jeweiliges Windelseitenteil vorteilhafterweise wie zuvor beschrieben seinerseits wenigstens um eine in Längsrichtung verlaufende Falzlinie auf sich selbst gefaltet sein. Vorzugsweise ist diese eingeschlagene Anordnung an einer ersten Fügestelle lösbar fixiert. Es wird diesbezüglich auf den Offenbarungsgehalt der DE102005035544.7 Bezug genommen.

Zum Schließen des Inkontinenzartikels im angelegten Zustand an einen Benutzer weisen die Windelseitenteile, vorzugsweise die hinteren Windelseitenteile Verschlusselemente auf, die mechanisch haftend oder klebend ausgebildet sein können, und die vorzugsweise ihrerseits in einer gefalteten, zum Gebrauch entfaltbaren Konfiguration an den Windelseitenteilen angeordnet sind. Es erweist sich als zweckmäßig, wenn die Verschlusselemente mit einem Auftreffbereich an der Außenseite des Windelhauptteils und/oder an der der Windelseitenteile lösbar haftend oder klebend zusammenwirken können.

Schließlich erweist es sich als vorteilhaft, wenn die vereinzelten Inkontinenzwegwerfwindeln um mindestens eine quer zur ersten Längsrichtung erstreckte Faltlinie gefaltet werden.

Es wird auch Schutz in Anspruch genommen für Inkontinenzwegwerfwindeln mit Merkmalen entsprechend oder gemäß den Merkmalen der beigefügten Verfahrensansprüche der vorliegenden Patentanmeldung.

Weitere Merkmale, Einzelheiten und Vorteile der Erfindung ergeben sich aus den beigefügten Patentansprüchen und aus der zeichnerischen Darstellung und nachfolgenden Beschreibung einer bevorzugten Ausführungsform des erfindungsgemäßen Verfahrens.

Es wird nachfolgend ein Test zur Bestimmung der Dehnbarkeit der beschrieben. Es wird unter Verwendung eines Zugprüfgeräts nach EN ISO 527-1 (April 1996) die Dehnung von Windelseitenteilen bis zu einer definierten Kraftgrenze im Zugversuch ermittelt.

### Probenvorbereitung:

Zunächst wird bei einer Inkontinenzwegwerfwindel, die zuvor über 24h bei 23 °C und 50% relativer Luftfeuchte konditioniert wurde, ein an den Windelhauptteil angefügtes Windelseitenteil entlang eines Seitenlängsrandes des Windelhauptteils unter Zerstörung der Anfügung abgetrennt. Hierfür kann eine Klinge oder Schere Verwendung finden. Sollte das Windelseitenteil an Fügestellen oder Fügebereichen lösbar fixiert in auf sich selbst gefalteter Konfiguration oder in einer übereinander eingeschlagenen Anordnung der Windelseitenteile vorliegen, werden vor der Abtrennung vom Windelhauptteil zunächst manuell sämtliche Fügestellen und Fügebereiche gelöst und das Windelseitenteil vollständig entfaltet. Das Windelseitenteil wird dann mit einem Längsseitenrand mittig zentriert an eine untere Klemme des Zugprüfgeräts über seine gesamte Länge (in Längsrichtung des Inkontinenzartikels), mit der er zuvor an den Windelhauptteil angefügt war, fest eingespannt (Einspanntiefe: 15mm). Die untere Klemme des Zugprüfgeräts muss deshalb eine entsprechende Länge, zweckmäßigerweise eine Länge von 300 mm aufweisen. Am gegenüberliegenden freien Längsseitenrand des abgetrennten Windelseitenteils wird die bewegbare Klemme des Zugprüfgeräts ebenfalls über seine gesamte Länge fest eingespannt (Einspanntiefe: 15mm). Die der Zugkraft auszusetzende Ausgangslänge entspricht somit der vollen Windelseitenteilbreite abzüglich der Einspanntiefe in die Klemmen. Durch gesteuerte Bewegung-dieser bewegbaren Klemme wird ein Zugprüfversuch bis zur definierten Kraftgrenze, insbesondere bis zu einer Kraft von 45 N durchgeführt und die Dehnung in % der Ausgangslänge ermittelt.

### Prüfparameter:

- Einspanntiefe: jeweils 15 mm
- Prüfgeschwindigkeit der bewegbaren Klemme: 300 mm/min
- Messweg: Dehnung bis zur Erreichung des definierten Kraftwertes von 45 N,
- Vorkraft: 0,2 N.

In der Zeichnung zeigt:
- Figur 1: eine schematische Darstellung des erfindungsgemäßen Herstellungsverfahrens;
- Figur 2: eine Draufsicht auf eine nach dem erfindungsgemäßen Verfahren hergestellte Inkontinenzwegwerfwindel in schematischer Darstellung;
- Figur 3: eine Draufsicht auf eine Windelseitenteilbahn in schematischer Darstellung mit angedeuteten Trennlinien;
- Figur 4: eine Draufsicht auf eine Windelseitenteilbahn in schematischer Darstellung mit angedeutetem Trennlinienverlauf (mit Schnittabfall);
- Figur 5: eine Draufsicht auf abgetrennte Längsabschnitte der Windelseitenteilbahn nach Figur 4;
- Figur 6: eine Draufsicht auf eine Windelseitenteilbahn nach einer weiteren erfindungsgemäßen Verfahrensvariante mit einem in Längsrichtung durchgehendem elastischem Material;
- Figur 7: eine Draufsicht auf eine Windelhauptteilbahn mit applizierten Längsabschnitten einer Windelseitenteilbahn;
- Figur 8: eine Draufsicht auf eine Windelhauptteilbahn mit applizierten Längsabschnitten einer Windelseitenteilbahn mit angedeutetem Konturschnitt zum Vereinzeln;
- Figur 9: eine Draufsicht auf eine Windelhauptteilbahn mit applizierten Längsabschnitten mit einem angedeuteten geraden Trennschnitt;
- Figur 10: eine Draufsicht auf eine Windelseitenteilbahn nach einer weiteren erfindungsgemäßen Verfahrensvariante;
- Figur 11: eine Draufsicht auf eine Windelseitenteilbahn nach einer weiteren erfindungsgemäßen Verfahrensvariante und
- Figur 12: eine Draufsicht auf eine nach dem erfindungsgemäßen Verfahren unter Bereitstellung einer nach Figur 11 hergestellten Windelseitenteilbahn hergestellte Inkontinenzwegwerfwindel in schematischer Darstellung.

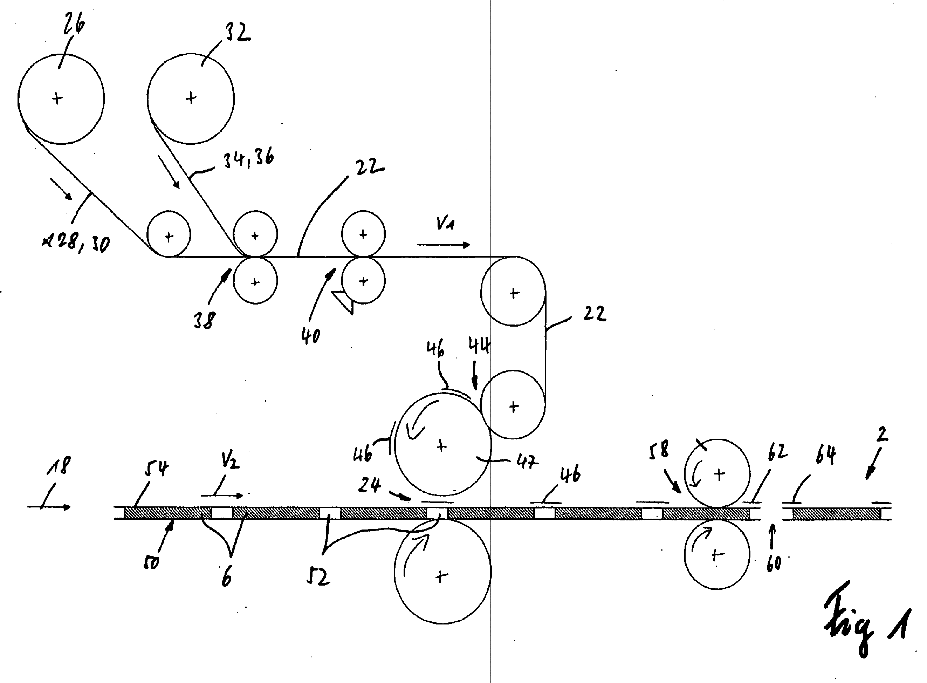

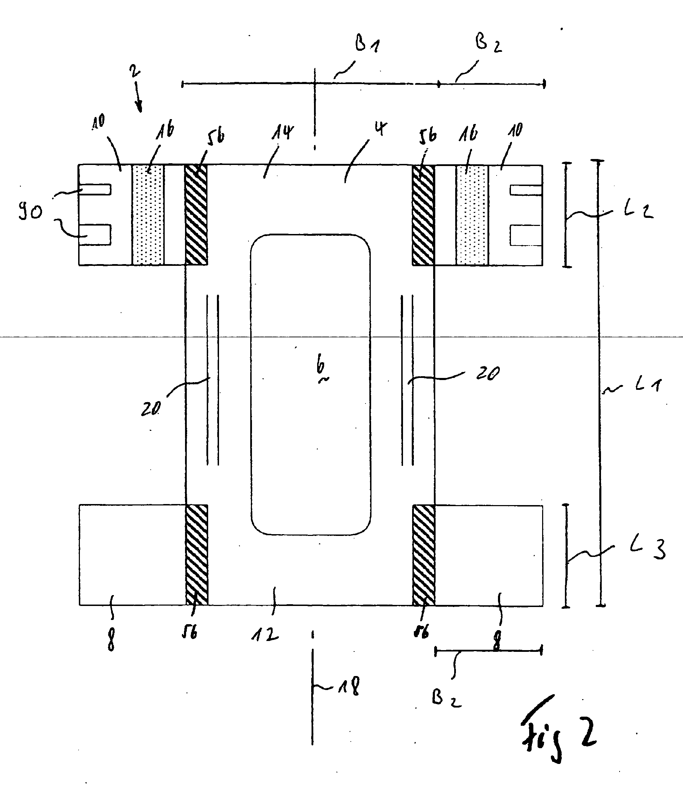

Figur 1 verdeutlicht das erfindungsgemäße Verfahren zum Herstellen einer Vielzahl von Inkontinenzwegwerfwindeln 2, die in der Draufsicht schematisch in Figur 2 dargestellt sind. Die herzustellenden Inkontinenzwegwerfwindeln 2 (im Folgenden als Windeln 2 bezeichnet) umfassen einen Windelhauptteil 4 mit einem darauf aufgebrachten Absorptionskern 6, dessen Längserstreckung geringer ist als die Längserstreckung des Windelhauptteils 4, und vordere Windelseitenteile 8 und hintere Windelseitenteile 10. Die Länge des Windelhauptteils L1 beträgt im in Fig. 2 dargestellten Fall 815 mm. Die Breite B1 des Windelhauptteils beträgt 320 mm. Die Länge L2 der hinteren Windelseitenteile beträgt 230 mm. Die Länge der vorderen Windelseitenteile L3 beträgt 170 mm. Die Breite B2 der vorderen und hinteren Windelseitenteile beträgt einheitlich 170 mm. Die vorderen und hinteren Windelseitenteile 8 und 10 sind an einem vorderen Hüftbereich 12 bzw. an einem hinteren Hüftbereich 14 des Windelhauptteils 4 unlösbar angefügt (im dargestellten Fall der Figur 2 jeweils seitlich). Die hinteren Windelseitenteile 10 weisen eine größere Dehnbarkeit auf als die vorderen Windelseitenteile 8, was durch einen elastisch dehnbaren Bereich oder Abschnitt 16 bei den hinteren Windelseitenteilen 10 schematisch angedeutet ist. Ferner dargestellt sind in einer Längsrichtung 18 der Windel 2 verlaufende erste elastische Elemente 20 beidseits des Absorptionskerns 6 angedeutet. Weitere hier nicht dargestellte zweite elastische Elemente können in Form aufstehender Cuff- oder Bündchenelemente vorliegen, die einen Seitenauslaufschutz im Wesentlichen bei den Rändern des Absorptionskerns 6 bilden. Erkennbar sind schematisch angedeutet an den hinteren Windelseitenteilen angebrachte Verschlusselemente 90 die in Gebrauch mit der Außenseite des Windelhauptteils und vorzugsweise auch mit der der vorderen Windelseitenteile haftend zusammenwirken können.

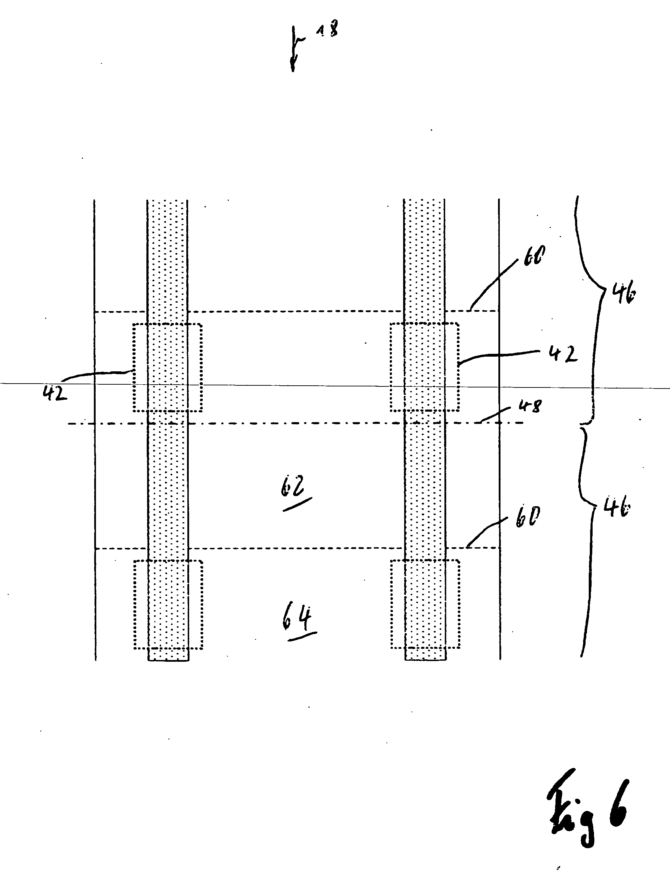

Zur Herstellung der Windel 2 wird gemäß Figur 1 (oberer Bereich) eine Windelseitenteilbahn 22 gebildet und in Richtung auf eine Applikationsstation 24 gefördert. Die Windelseitenteilbahn 22 umfasst eine erste von einer Vorratsrolle 26 endlos abrollbare erste Bahn 28 aus einem im Wesentlichen insbesondere undehnbaren Trägermaterial 30 und eine von einer Vorratsrolle 32 endlos abwickelbare zweite Bahn 34 aus einem elastisch dehnbaren Material 36. Die in Figur 1 nur schematisch dargestellte zweite Bahn 34 kann beispielsweise zwei streifenförmige in Querrichtung voneinander beabstandete Bahnen umfassen, so wie dies später in Verbindung mit Figur 6 dargestellt ist. Die Bahnen 28 und 34 werden übereinander gefördert und durch an sich beliebige Fügetechniken miteinander dauerhaft verbunden, was durch zwei Rollen, die eine Fügestation 38 bilden, angedeutet ist. Der Fügestation 38 folgt in Förderrichtung eine Aktivierungsstation 40, in der die elastisch dehnbaren Eigenschaften des elastisch dehnbaren Materials 36 aktiviert werden, und zwar dadurch, dass bereichsweise - wie dies in Figur 6 angedeutet ist das nicht dehnbare Trägermaterial 30 der ersten Bahn 28 in einem Bereich 42 (s. Figur 6) derart behandelt wird, dass es einer Dehnung nicht mehr zu widerstehen vermag. Dies kann beispielsweise durch eine bereichsweise Überdehnung durch den Prozess des "ring rolling" erreicht werden, wo das aus erster Bahn 28 und zweiter Bahn 34 gebildete Verbundmaterial im Bereich 42 durch miteinander kämmende Walzenoberflächen derart gedehnt wird, das an sich nicht dehnbare Trägermaterial 30 überdehnt und dabei plastisch verformt wird. (Während das elastisch dehnbare Material 36 der Dehnung elastisch folgt.) Nach Durchlaufen der Aktivierungsstation 40 ist die Windelseiteriteilbahn 22 jedenfalls in dem in Figur 6 angedeuteten Bereich 42 elastisch dehnbar. In den übrigen Bereichen außerhalb des Bereichs 42 vermag das streifenförmig aufgebrachte elastisch dehnbare Material 36 seine elastifizierende Wirkung nicht zu entfalten, da das nicht dehnbare Trägermaterial 30 eine wesentliche Dehnung der Windelseitenteilbahn 22 verhindert.

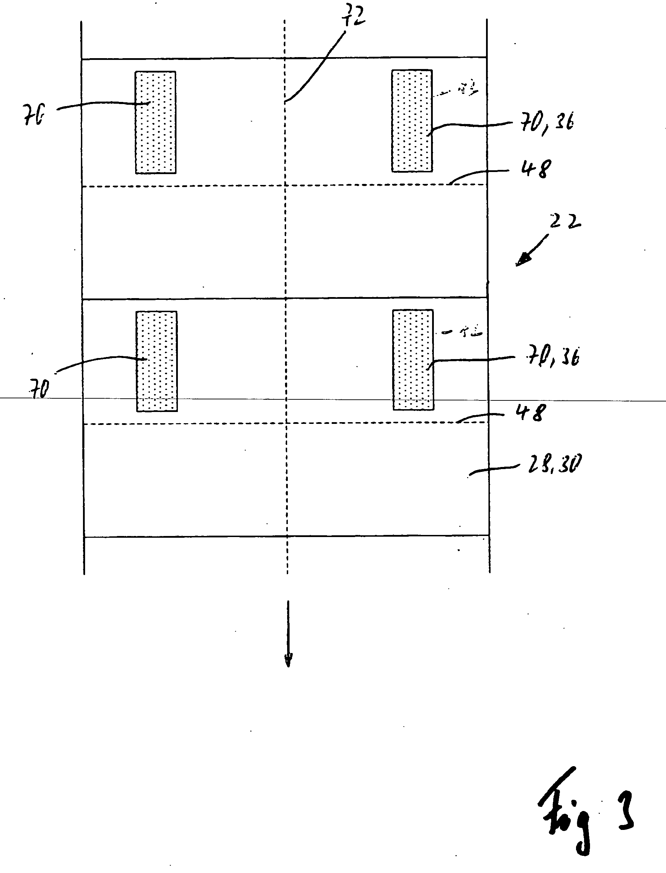

Die so behandelte Windelseitenteilbahn wird weiter mit der Geschwindigkeit v1 in Richtung auf eine Trennstation 44 gefördert, wo Längsabschnitte 46 quer zur Förderrichtung von der Windelseitenteilbahn 22 abgetrennt und über eine schematisch angedeutete Beschleunigungswalze 47 auf eine Geschwindigkeit v2 beschleunigt und der eingangs bereits erwähnten Applikationsstation 24 zugeführt werden. Diese Längsabschnitte 46 werden in den Figuren 3 bis 10 jeweils durch eine Trennlinie mit Bezugszeichen 48 begrenzt.

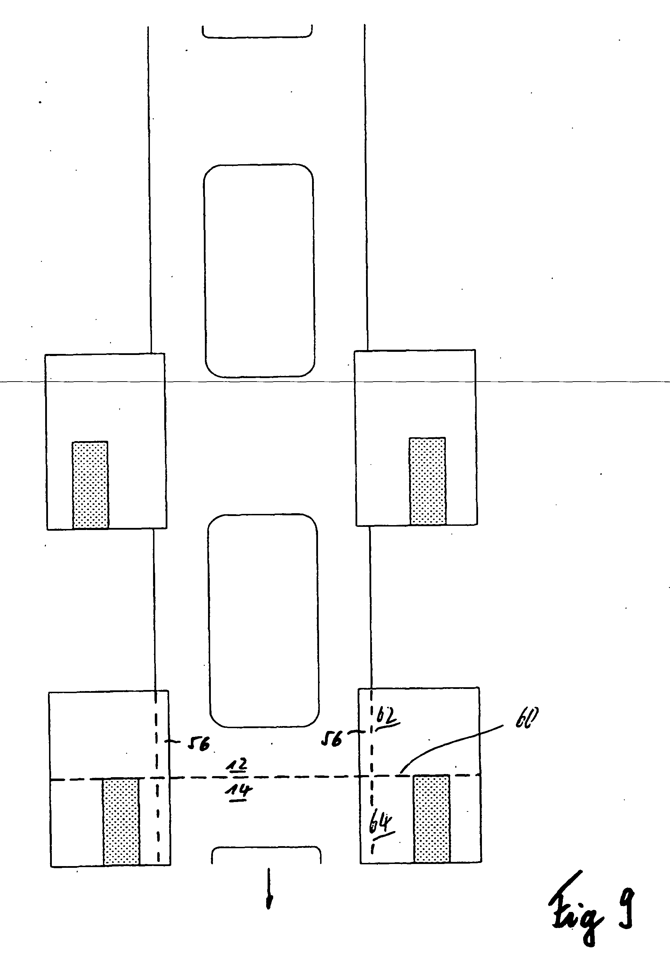

In Figur 1 unten ist das Zuführen und Fördern einer endlosen Windelhauptteilbahn 50 dargestellt. Die Windelhauptteilbahn trägt eine endlose Anzahl von aufeinanderfolgend abgelegten und voneinander beabstandeten Absorptionskernen 6. Des Weiteren angedeutet ist eine Decklage 54. Absorptionskern 6 und Decklage 54 werden im Folgenden als zu der Windelhauptteilbahn 50 gehörig angesehen. Die Windelhauptteilbahn wird in Richtung auf die Applikationsstation 24 mit der Geschwindigkeit v2 endlos gefördert. In der Applikationsstation 24 werden die Längsabschnitte 46 der Windelseitenteilbahn 22, wie aus Figur 1 ersichtlich, auf die Windelhauptteilbahn 50 appliziert, und zwar derart, dass sie den Bereich 52 zwischen zwei Absorptionskernen 6 überfangen oder in diesem Bereich angeordnet werden. Dies ist beispielsweise in der Draufsicht aus den Figuren 8 und 9 ersichtlich. In der Applikationsstation 24 oder vorzugsweise im Wesentlichen daran anschließend werden die aufgebrachten Längsabschnitte 46 unlösbar mit der Windelhauptteilbahn 50 verbunden, also daran fixiert. Ein solcher fixierter Bereich ist beispielsweise in Figuren 2, 8 und 9 jeweils mit dem Bezugszeichen 56 bezeichnet. Der so erhaltene Verbund wird weiter in Richtung auf eine vereinzelungsstation 58 gefördert, wo im Wesentlichen quer zur Förderrichtung, die der Längsrichtung 18 der herzustellenden Windeln 2 entspricht, ein Trennschnitt ausgeführt wird, beispielsweise mit einer rotierenden Messerwalze oder mit einem Stanzwerkzeug. Der Trennschnitt ist in den Figuren mit dem Bezugszeichen 60 angedeutet. Er wird so ausgeführt, dass er jeweils durch den applizierten Längsabschnitt 46 verläuft und diesen in einen ersten Teilabschnitt 62 und in einen zweiten Teilabschnitt 64 teilt. Der zweite Teilabschnitt bildet die im hinteren Hüftbereich 14 vorgesehenen Windelseitenteile 10 und der erste Teilabschnitt 62 bildet die im vorderen Hüftbereich 12 vorgesehenen Windelseitenteile 8.

Verschiedene erfindungsgemäße Verfahrensvarianten werden nun anhand der Figuren 3 bis 11 beschrieben:

Nach der in Figur 3 dargestellten Verfahrensvariante wird eine Windelseitenteilbahn 22 in Pfeilrichtung gefördert. Man erkennt die erste Bahn 28 aus Trägermaterial 30 mit streifenförmigen Abschnitten 70 von elastisch dehnbarem Material 36. Im Unterschied zur schematischen Andeutung der Figur 1 wurde das elastische Material 36 also nicht in der Förderrichtung kontinuierlich (so wie in Figur 6 dargestellt), sondern in voneinander beabstandeten Abschnitten 70 auf die erste Bahn 28 aufgebracht. Wiederum ist das Trägermaterial 30 der ersten Bahn 28 im Wesentlichen undehnbar. Um im Bereich 42 der streifenförmigen Abschnitte 70 eine Dehnbarkeit zu erreichen, muss das unelastische Trägermaterial 30 in diesen Bereichen, so wie vorausgehend beschrieben, behandelt werden. Insbesondere kann dort eine lokale Überdehnung des Trägermaterials 30 ausgeführt werden, oder es wäre denkbar, dass dort Ausnehmungen in dem Trägermaterial 30 vorgesehen werden.

Wie durch die zentrale gestrichelte Linie 72 angedeutet, wird die Windelseitenteilbahn 22 in der Längsrichtung in eine linke und rechte Bahn geteilt, die dann, wie in Figur 2 angedeutet, an seitliche Bereiche der Windelhauptteilbahn 4 angefügt werden.

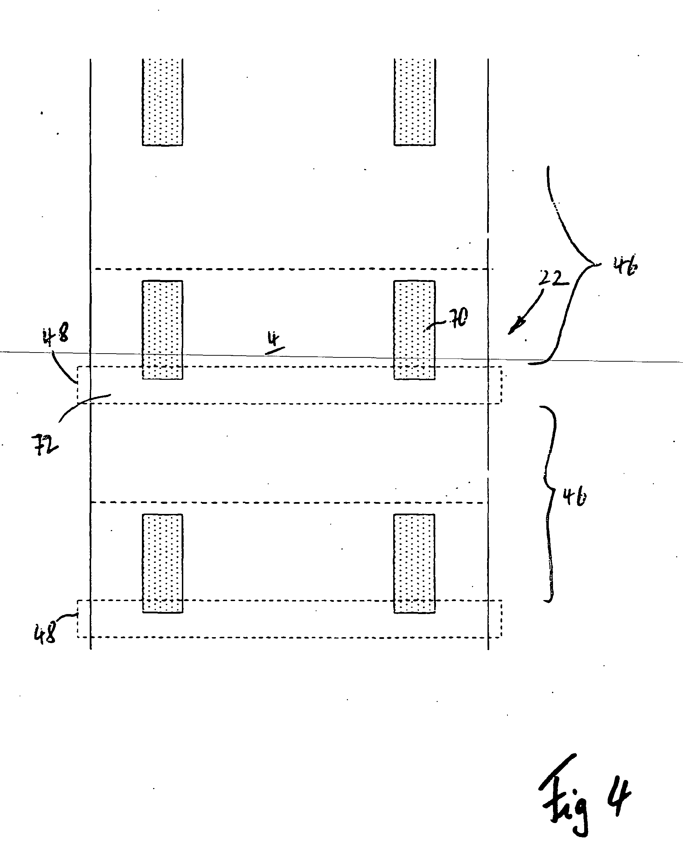

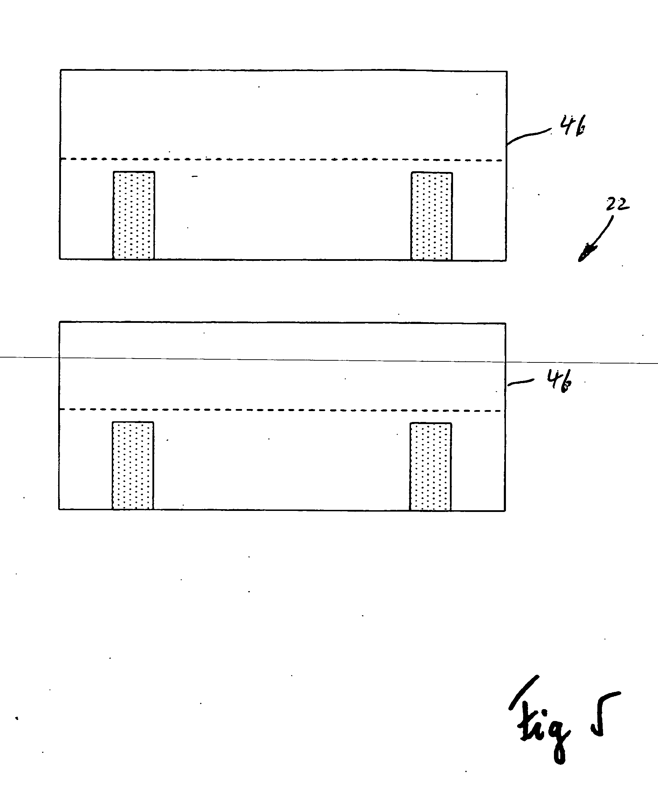

Figur 4 verdeutlicht den Verlauf einer Trennlinie 48 zur Bildung von Längsabschnitten 46 in der Trennstation 44. Die Trennlinie 48 wird rechteckförmig geführt, so dass ein rechteckförmiger zu verwerfender Abschnitt 72 aus der Windelseitenteilbahn 22 herausgetrennt wird. Die Trennlinie 48 wird dabei so geführt, dass sie das Ende eines jeweiligen streifenförmigen Abschnitts 70 aus elastisch dehnbarem Material 36 erfasst. Auf diese Weise können Positionsungenauigkeiten ausgeglichen werden, und es wird sichergestellt, dass sich ein jeweiliger streifenförmiger Abschnitt 70 bündig bis zum hinteren Hüftrand der Windel 2 bzw. des betreffenden Windelhauptteils 4 erstreckt. Figur 5 zeigt die abgetrennten Längsabschnitte 46 der Windelseitenteilbahn 22. Es sei an dieser Stelle ausdrücklich darauf hingewiesen, dass es sich auch als vorteilhaft erweisen kann, wenn beim Vereinzeln der Windel 2 in der Vereinzelungsstation 58 ein zu verwerfender Abschnitt beim Trennen gebildet wird. Auf diese Weise kann dann nämlich nicht nur erfindungsgemäß erreicht werden, dass sich der streifenförmige Abschnitt 70 aus elastisch dehnbarem Material bis zum hinteren Hüftrand erstreckt, sondern es kann auch sichergestellt werden, dass sich kein elastisches Material in den vorderen Hüftbereich 12 der angrenzenden Windel 4 bzw. des angrenzenden Windelhauptteils 4 hineinerstreckt. Dies ist im Zusammenhang mit Figur 9 verdeutlicht und wird nachfolgend erörtert werden.

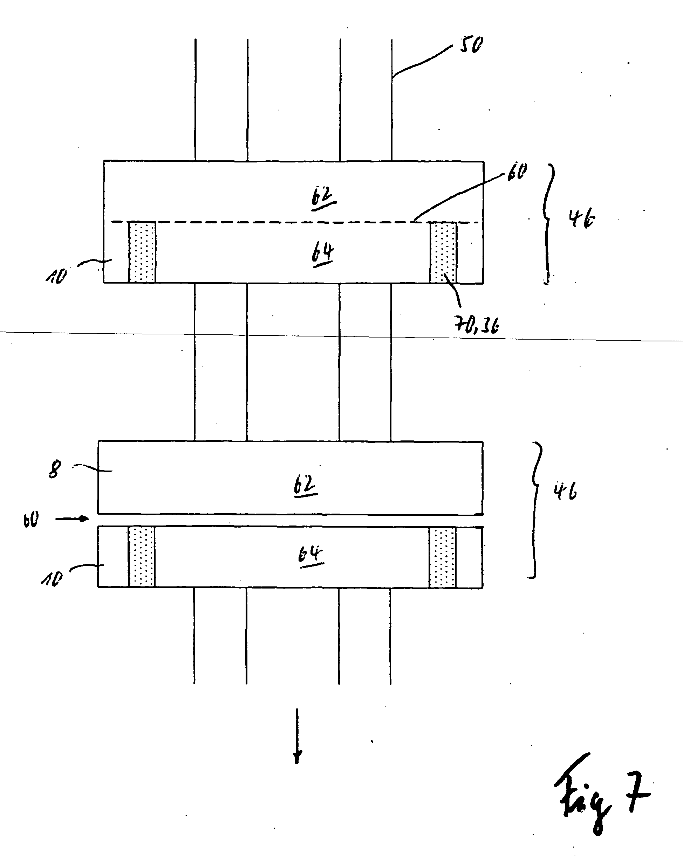

Anstelle der Bildung eines zu verwerfenden Abschnitts 72 beim Abtrennen der Längsabschnitte 46 von der Windelseitenteilbahn 22 könnte auch entlang einer einzigen Trennlinie 48 getrennt werden, so wie dies in Figuren 3 und 6 angedeutet ist. Figur 7 zeigt schematisch eine Windelhauptteilbahn 50, auf die voneinander beabstandete Längsabschnitte 46 einer Windelseitenteilbahn 22 aufgebracht und beispielsweise durch Ultraschallschweißen, durch Kleber oder in sonstiger Weise unlösbar mit der Windelhauptteilbahn 50 verbunden worden sind. In Figur 7 oben ist ein Trennschnitt 60 zum Vereinzeln der Windeln 2 lediglich angedeutet, während in Figur 7 unten die Vereinzelungsstation 58 bereits durchlaufen wurde und der Trennschnitt 60 ausgeführt wurde. Dabei wird ein erster Teilabschnitt 62 gebildet, der kein elastisch dehnbares Material 36 aufweist, und ein zweiter Teilabschnitt 64, der den streifenförmigen Abschnitt oder Bereich 70 aus elastisch dehnbarem Material 36 aufweist. Der erste Teilabschnitt 62 bildet vordere Windelseitenteile 8, und der hintere Teilabschnitt 64 bildet hintere Windelseitenteile 10. Im beispielhaft dargestellten Fall der Figur 7 wurde die Windelseitenteilbahn 22 nicht entlang ihrer Längsrichtung in einen linken und einen rechten Teil getrennt, sondern ein jeweils gebildeter Längsabschnitt 46 erstreckt sich in Querrichtung der Windel 2 einstückig durchgehend über die Breite der Windel; er überfängt also die Windel in Querrichtung durchgehend (im Unterschied zur schematischen Darstellung der Figur 2).

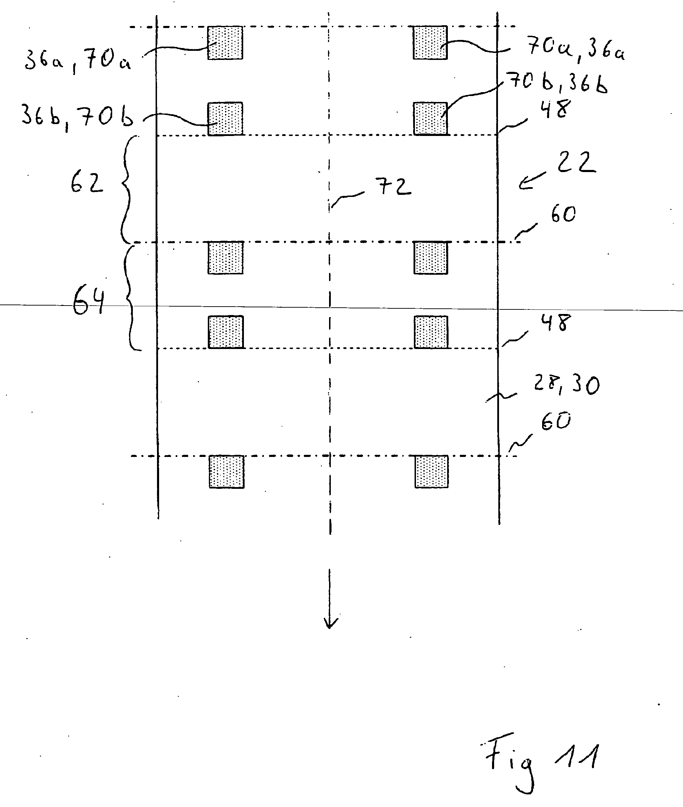

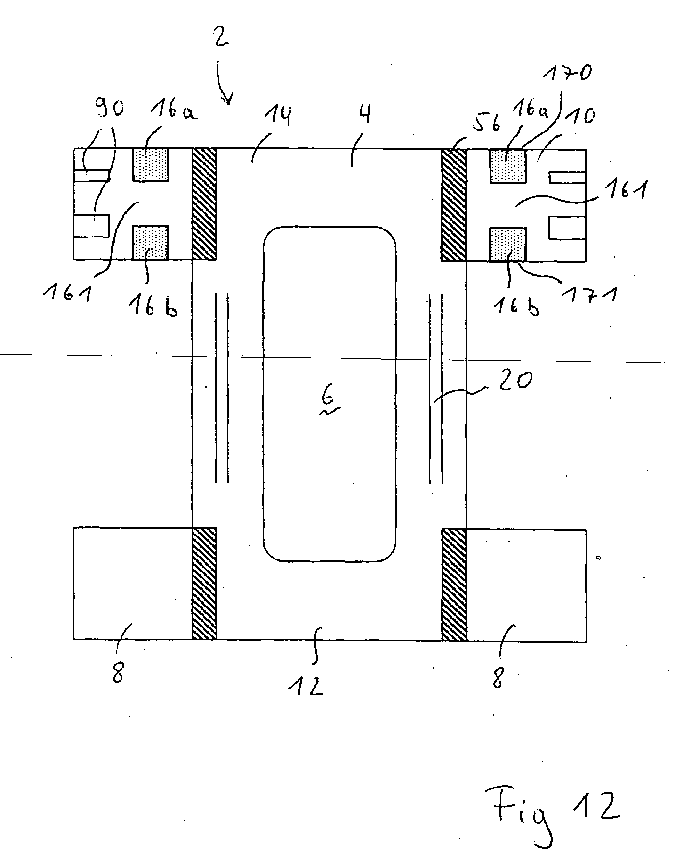

Nach der in Figur 11 dargestellten Verfahrensvariante wird eine Windelseitenteilbahn 22 in Pfeilrichtung gefördert. Man erkennt die erste Bahn 28 aus Trägermaterial 30 mit streifenförmigen Abschnitten 70a, 70b von elastisch dehnbarem Material 36a, 36b. Im Unterschied zur schematischen Andeutung der Figur 3 sind in einem jeweiligen zweiten Teilabschnitt 64, welcher ein hinteres Windelseitenteil bildet, jeweils zwei streifenförmige Abschnitte 70a, 70b von elastisch dehnbarem Material 36a, 36b vorgesehen. In einem jeweiligen ersten Teilabschnitt 62, welcher ein vorderes Windelseitenteil bildet, ist kein elastisch dehnbarer Bereich vorgesehen. Dies hat, wie Figur 12 veranschaulicht zu Folge, dass ein jeweiliges hinteres Windelseitenteil 10 am oder in der Nähe eines oberen Querrandes 170 des Windelseitenteils einen ersten dehnbaren Bereich 16a und am oder in der Nähe eines unteren Querrandes 171 einen zweiten dehnbaren Bereich 16b aufweist. Zwischen den dehnbaren Bereichen 16a, 16b verbleibt ein im Wesentlichen undehnbarer Bereich 161. Solchenfalls ist das hintere Windelseitenteil genau dort mit einer höheren Dehnbarkeit ausgestattet, wo erfahrungsgemäß in Gebrauch, beispielsweise beim Schließen der Inkontinenzwegwerfwindel, die höchsten Kräfte einwirken. Einem Abreißen der Windelseitenteile vom Hauptteil kann hierdurch vorgebeugt werden. Der undehnbare Bereich 161 gewährleistet außerdem eine höhere Stabilität des Windelseitenteils, was sowohl der Handhabung der Inkontinenzwegwerfwindel in Gebrauch als auch deren Herstellung in der schnell laufenden Windelmaschine zugute kommt.

Es versteht sich, dass auch die nach Figur 11 diskontinuierlich angeordneten elastisch dehnbaren Bereiche wie vorstehend beschrieben durch lediglich getaktete Aktivierung von zuvor in Förderrichtung kontinuierlich aufgebrachtem elastischen Material in der zuvor beschriebenen Weise vorgesehen werden können.

Figur 8 zeigt eine andere Ausführungsform, bei der die Windelseitenteilbahn 22 in Längsrichtung in einen linken und einen rechten Teil getrennt wurde. Dies geschieht vorteilhafterweise, aber nicht notwendigerweise, vor der Trennstation 44, wo die Längsabschnitte 46 gebildet werden. Entsprechend wird ein linker und ein rechter Längsabschnitt 46 auf die Windelhauptteilbahn 50 in der Applikationsstation 24 aufgebracht und unlösbar fixiert. Ebenfalls abgedeutet ist der Verlauf eines als Konturschnitt 78 ausgebildeten Trennschnitts 60, bei dessen Ausführung wiederum ein zu verwerfender Abschnitt 80 gebildet wird. Wie bereits im Zusammenhang mit Figur 4 erläutert, wird einerseits erreicht, dass sich der streifenförmige Bereich 70 aus elastischem Material 36 bündig bis zum hinteren Hüftrand der vereinzelten Windel 2 erstreckt, und andererseits wird verhindert, dass elastisches Material 36 in den ersten Teilabschnitt 62 gelangt. Die Ausführung eines einzigen insbesondere geraden Trennschnitts 60 ist in Figur 9 angedeutet.

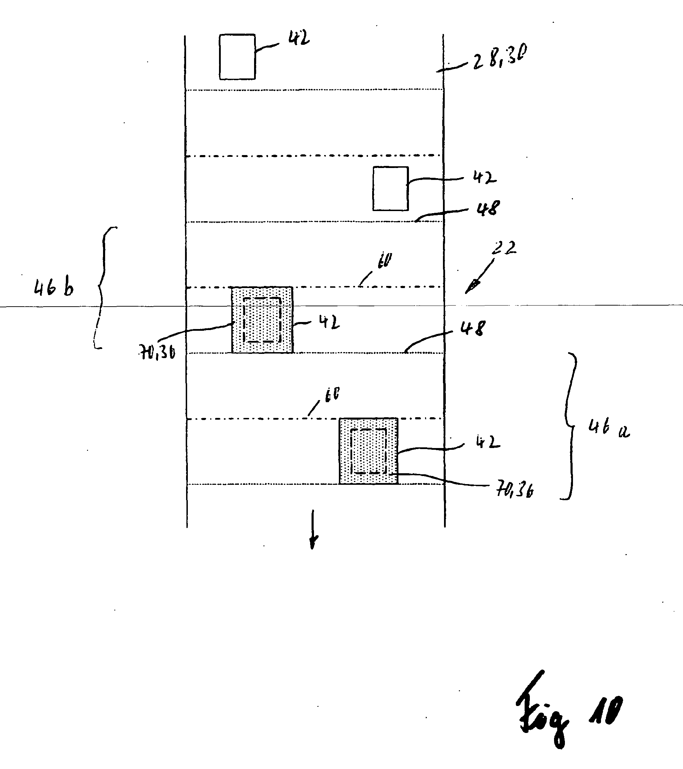

Figur 10 zeigt eine weitere alternative erfindungsgemäße Verfahrensvariante, bei der jeweils die gesamte Breite der zur Verfügung stehenden Windelseitenteilbahn 22 als linkes oder rechtes Windelseitenteil verwendet wird. Wiederum angedeutet ist ein Bereich 42 in der ersten Bahn 28 aus Trägermaterial 30, in dem das nicht elastische Trägermaterial 30 derart behandelt wird, dass es einer Dehnung keinen oder einen geringen Widerstand leistet. Insbesondere könnte der Bereich 42 einen durch "ring rolling" überdehnten Bereich bezeichnen. Der jeweilige Bereich 42 ist, wie in Förderrichtung nachfolgend dargestellt, mit einem Abschnitt oder Bereich 70 aus elastisch dehnbarem Material 36 überfangen. Die Besonderheit der in Figur 10 dargestellten Verfahrensvariante liegt darin, dass alternierend ein rechtes Windelseitenteil und ein linkes Windelseitenteil gebildet wird. Dies bedeutet, dass in der Trennstation 44 ein erster Längsabschnitt 46a und daran anschließend ein zweiter Längsabschnitt 46b abgetrennt wird, die auf die eine und auf die andere Seite der Windelhauptteilbahn 50 angefügt werden. Ein jeweiliger Trennschnitt 60 zur Vereinzelung der Windeln ändert sich hierbei nicht grundsätzlich. Nach dieser Verfahrensvariante kann, wie erwähnt, eine größere Breite der Windelseitenteile 8, 10 realisiert werden.

Es wird ausdrücklich darauf hingewiesen, dass die Windelseitenteile 8, 10 auch in auf sich selbst gefalteter Konfiguration vorliegen können, wie dies DE202004006951.2 offenbart und lehrt. Vorzugsweise erfolgt dieses Einfalten bzw. auf sich selbst Falten entlang einer in Längsrichtung erstreckten Faltlinie vor der Trennstation 44, wo die Längsabschnitte 46 gebildet werden. Die aufeinander gefalteten Abschnitte werden vorzugsweise lösbar, insbesondere durch Ultraschallschweißpunkte oder durch schwach wirkende Kleberstellen aufeinander fixiert, so dass sie leicht wieder manuell entfaltbar sind.

## Patentansprüche

1. Verfahren zum Herstellen einer Vielzahl von einen Windelhauptteil (4) und daran angefügte vordere und hintere Windelseitenteile (8, 10) aufweisenden Inkontinenzwegwerfwindeln (2), **gekennzeichnet durch** folgende Verfahrensschritte:
- Zuführen und Fördern einer endlosen Windelhauptteilbahn (50) in einer ersten Längsrichtung (18) zu einer Applikationsstation (24),
- Zuführen einer endlosen Windelseitenteilbahn (22) mit einem ersten endlosen Trägermaterial (30), in der gegenüber dem ersten Trägermaterial (30) dehnbare Bereiche (70) in der Längsrichtung der Windelseitenteilbahn (22) alternierend, das heißt mit einem Abstand voneinander, vorgesehen werden,
- Abtrennen von Längsabschnitten (46) der Windelseitenteilbahn (22) und Zuführen der Längsabschnitte (46) zu der Applikationsstation (24) und Positionieren an der Windelhauptteilbahn (50),
- Unlösbares Fixieren der Längsabschnitte (46) der Windelseitenteilbahn (22) an ersten Bereichen (56) der Windelhauptteilbahn, wobei die ersten Bereiche (56) jeweils einen vorderen Hüftbereich (12) und einen hinteren Hüftbereich (14) zweier in Längsrichtung aufeinanderfolgender und aneinander angrenzender Windelhauptteile (4) umfassen,
- Vereinzeln der Inkontinenzwegwerfwindeln (2) **durch** Trennen der Windelhauptteilbahn (50) quer zur
Längsrichtung (18), wobei das Trennen **durch** die Längsabschnitte (46) hindurchgeführt wird, derart dass ein erster Teilabschnitt (62) eines jeweiligen Längsabschnitts (46) ein vorderes Windelseitenteil (8) einer ersten Inkontinenzwegwerfwindel bildet und ein zweiter Teilabschnitt (64) des jeweiligen Längsabschnitts (46) ein hinteres Windelseitenteil (10) einer unmittelbar angrenzenden zweiten Inkontinenzwegwerfwindel bildet,
- wobei das hintere Windelseitenteil (10) eine höhere Dehnbarkeit aufweist als das vordere Windelseitenteil (8).

2. Verfahren nach Anspruch 1, **dadurch gekennzeichnet, dass** ein jeweiliger Windelhauptteil (4) einen Flüssigkeiten speichernden Absorptionskern (6) aufweist.

3. Verfahren nach Anspruch 1 oder 2, **dadurch gekennzeichnet, dass** die dehnbaren Bereiche (70) elastisch dehnbare Bereiche sind.

4. Verfahren nach einem der vorstehenden Ansprüche, **dadurch gekennzeichnet, dass** in einem zweiten Teilabschnitt (64) der Windelseitenteilbahn (22) in der Längsrichtung (18) voneinander beabstandet zumindest zwei dehnbare Bereiche (70a, 70b) vorgesehen werden.

5. Verfahren nach einem der vorstehenden Ansprüche , **dadurch gekennzeichnet, dass** in der ersten Längsrichtung (18) erstreckte erste elastische Elemente (20) an die Windelhauptteilbahn (50)angefügt werden.

6. Verfahren nach einem der vorstehenden Ansprüche, **dadurch gekennzeichnet, dass** in der ersten Längsrichtung (18) erstreckte zweite elastische Elemente an die Windelhauptteilbahn (50) angefügt werden.

7. Verfahren nach einem der vorstehenden Ansprüche, **dadurch gekennzeichnet, dass** die zweiten elastischen Elemente durch aufstehende Cuff-Elemente gebildet werden.

8. Verfahren nach einem der vorstehenden Ansprüche, **dadurch gekennzeichnet, dass** das Zuführen der Windelseitenteilbahn (22) zu der Applikationsstation (24) mit einer ersten Geschwindigkeit v1 und das Zuführen der endlosen Windelhauptteilbahn (50) zu der Applikationsstation (24) mit einer zweiten Geschwindigkeit v2 erfolgt, wobei die erste Geschwindigkeit v1 geringer ist als die zweite Geschwindigkeit v2.

9. Verfahren nach Anspruch 8, **dadurch gekennzeichnet, dass** die zweite Geschwindigkeit v2 um mindestens 40% insbesondere um mindestens 70%, insbesondere um mindestens 90% insbesondere um höchstens 200 % größer ist als die erste Geschwindigkeit v1.

10. Verfahren nach einem der vorstehenden Ansprüche, **dadurch gekennzeichnet, dass** die elastischen Bereiche (70) der Windelseitenteilbahn (22) durch Aufbringen von elastischem Material (36) auf das Tragermaterial (30) und bereichsweises Überdehnen des Trägermaterials (30) gebildet werden.

11. Verfahren nach einem der vorstehenden Ansprüche, **dadurch gekennzeichnet, dass** das elastische Material der Windelseitenteilbahn (22) von in der Längsrichtung der Windelseitenteilbahn kontinuierlich erstrecktem, insbesondere streifenförmigem elastischen Material (36) gebildet ist, das aber in der Längsrichtung nur abschnittsweise aktiviert wird.

12. Verfahren nach einem der vorstehenden Ansprüche, **dadurch gekennzeichnet, dass** das elastische Material (36) sandwichartig zwischen einem ersten und einem zweiten Trägermaterial angeordnet ist.

13. Verfahren nach einem der vorstehenden Ansprüche, **dadurch gekennzeichnet, dass** ein Längsabschnitt (46) der Windelseitenteilbahn (22) in einer Querrichtung der Windel (2) einstückig durchgehend ausgebildet wird, so dass er den betreffenden vorderen oder hinteren Hüftbereich (12, 14) des Windelhauptteils (4) durchgehend überfängt.

14. Verfahren nach einem der vorstehenden Ansprüche, **dadurch gekennzeichnet, dass** bei der Windelseitenteilbahn (22) in Längsrichtung aufeinanderfolgend ein Längsabschnitt (46a) für die Bildung eines rechten Windelseitenteils und ein Längsabschnitt (46b) für die Bildung eines linken Windelseitenteils vorgesehen werden.

15. Verfahren nach einem der vorstehenden Ansprüche, **dadurch gekennzeichnet, dass** beim Vereinzeln der Inkontinenzwegwerfwindeln (2) oder beim Abtrennen der Längsabschnitte (46) ein zu verwerfender Abschnitt (72, 80) gebildet wird.

16. Verfahren nach einem der vorstehenden Ansprüche, **dadurch gekennzeichnet, dass** beim Vereinzeln ein Konturschnitt (78) ausgeführt wird.

17. Verfahren nach einem der vorstehenden Ansprüche, **dadurch gekennzeichnet, dass** das hintere Windelseitenteil bei einer Krafteinwirkung von 45 N eine Dehnung von mindestens 20%, insbesondere mindestens 25% und weiter insbesondere mindestens 30% aufweist.

18. Verfahren nach einem der vorstehenden Ansprüche, **dadurch gekennzeichnet dass** das vordere Windelseitenteil bei einer Krafteinwirkung von 45 N eine Dehnung von höchstens 15%, insbesondere höchstens 10% und weiter insbesondere von höchstens 8% aufweist.

19. Verfahren nach einem der vorstehenden Ansprüche, **dadurch gekennzeichnet, dass** die vorderen Windelseitenteile (8) im Wesentlichen undehnbar ausgebildet werden.

20. Verfahren nach einem der vorstehenden Ansprüche, **dadurch gekennzeichnet, dass** die Windelseitenteile (8, 10) um mindestens eine, insbesondere zwei in der ersten Längsrichtung (18) erstreckte Faltlinien gefaltet werden.

21. Verfahren nach einem der vorstehenden Ansprüche, **dadurch gekennzeichnet, dass** die Windelseitenteile (8, 10) auf sich selbst gefaltet werden und in dieser Konfiguration lösbar fixiert werden.

22. Verfahren nach einem der vorstehenden Ansprüche, **dadurch gekennzeichnet, dass** die vereinzelten Inkontinenzwegwerfwindeln (2) um mindestens eine, insbesondere zwei quer zur ersten Längsrichtung (18) erstreckte Faltlinien gefaltet werden.

## Claims

1. Method for producing a plurality of disposable incontinence diapers (2) comprising a diaper main part (4) and front and rear diaper side parts (8, 10) joined thereto, **characterized by** the following method steps:
- supplying and delivering an endless diaper main part sheet (50) in a first longitudinal direction (18) to an application station (24),
- supplying an endless diaper side part sheet (22) with a first endless carrier material (30), wherein expandable areas (70) are provided within the endless diaper side part sheet (22), which are expandable versus the first endless carrier material (30) and which are alternatingly provided in the longitudinal direction, which means with a separation therebetween,
- separating longitudinal sections (46) of the diaper side part sheet (22), supplying the longitudinal sections (46) to the application station (24), and positioning them on the diaper main part sheet (50),
- undetachably fixing the longitudinal sections (46) of the diaper side part sheet (22) to first areas (56) of the diaper main part sheet, wherein each first area (56) comprises a front hip area (12) and a rear hip area (14) of two diaper main parts (4) which are disposed one after the other in the longitudinal direction and border each other,
- singling the disposable incontinence diapers (2) through separating the diaper main part sheet (50) transversely to the longitudinal direction (18), wherein separation is performed through the longitudinal sections (46), such that a first partial section (62) of a respective longitudinal section (46) forms a front diaper side part (8) of a first disposable incontinence diaper and a second partial section (64) of the respective longitudinal section (46) forms a rear diaper side part (10) of a directly bordering second disposable incontinence diaper,
- wherein the rear diaper side part (10) has a greater expansibility than the front diaper side part (8).

2. Method according to claim 1, **characterized in that** a respective diaper main part (4) has an absorption core (6) that stores liquids.

3. Method according to claim 1 or 2, **characterized in that** the expandable areas (70) are elastically expandable areas.

4. Method according to any one of the preceding claims, **characterized in that** at least two expandable areas (70a, 70b) are provided in a second partial section (64) of the diaper side part sheet (22), which are spaced apart in the longitudinal direction (18).

5. Method according to any one of the preceding claims, **characterized in that** first elastic elements (20), which extend in the first longitudinal direction (18), are joined to the diaper main part sheet (50).

6. Method according to any one of the preceding claims, **characterized in that** second elastic elements, which extend in the first longitudinal direction (18), are joined to the diaper main part sheet (50).

7. Method according to any one of the preceding claims, **characterized in that** the second elastic elements are formed by raising cuff elements.

8. Method according to any one of the preceding claims, **characterized in that** the diaper side part sheet (22) is supplied to the application station (24) at a first speed v1 and the endless diaper main part sheet (50) is supplied to the application station (24) at a second speed v2, wherein the first speed v1 is slower than the second speed v2.

9. Method according to claim 8, **characterized in that** the second speed v2 is faster than the first speed v1 by at least 40%, in particular at least 70%, in particular at least 90%, in particular at most 200%.

10. Method according to any one of the preceding claims, **characterized in that** the elastic areas (70) of the diaper side part sheet (22) are formed by disposing elastic material (36) onto the carrier material (30) and overstretching the carrier material (30) in some areas.

11. Method according to any one of the preceding claims, **characterized in that** the elastic material of the diaper side part sheet (22) is formed, in particular, by a lamellar elastic material (36) that extends continuously in the longitudinal direction of the diaper side part sheet and is activated only in sections in the longitudinal direction.

12. Method according to any one of the preceding claims, **characterized in that** the elastic material (36) is disposed like a sandwich between a first and a second carrier material.

13. Method according to any one of the preceding claims, **characterized in that** a longitudinal section (46) of the diaper side part sheet (22) is formed continuously in one piece in a transverse direction of the diaper (2), such that it continuously bridges the respective front or rear hip area (12, 14) of the diaper main part (4).

14. Method according to any one of the preceding claims, **characterized in that** in a longitudinal direction of the diaper side part sheet (22), one longitudinal section (46a) is provided for forming a right-hand diaper side part and subsequent thereto a longitudinal section (46b) is provided for forming a left-hand diaper side part.

15. Method according to any one of the preceding claims, **characterized in that** a section (72, 80) to be discarded is formed during singling of the disposable incontinence diapers (2) or during separation of the longitudinal sections (46).

16. Method according to any one of the preceding claims, **characterized in that** a contour cut (78) is performed during singling.

17. Method according to any one of the preceding claims, **characterized in that** the rear diaper side part has an expansibility of at least 20%, in particular at least 25 % and moreover, in particular, at least 30 % under the action of a force of 45N.

18. Method according to any one of the preceding claims, **characterized in that** the front diaper side part has an expansibility of at most 15 %, in particular at most 10% and moreover, in particular, at most 8 % under the action of a force of 45N.

19. Method according to any one of the preceding claims, **characterized in that** the front diaper side parts (8) are substantially non-expandable.

20. Method according to any one of the preceding claims, **characterized in that** the diaper side parts (8, 10) are folded about at least one, in particular, two folding lines that extend in the first longitudinal direction (18).

21. Method according to any one of the preceding claims, **characterized in that** the diaper side parts (8, 10) are folded on top of each other and are detachably fixed in this configuration.

22. Method according to any one of the preceding claims, **characterized in that** the singled disposable incontinence diapers (2) are folded about at least one, in particular, two folding lines that extend transversely to the first longitudinal direction (18).

## Revendications

1. Procédé pour fabriquer une pluralité de couches jetables pour personnes incontinentes (2) présentant une partie principale de couche (4) et des parties latérales de couche avant et arrière (8, 10) jointes à cette dernière, **caractérisé par** les étapes consistant à :
- amener et transporter une bande de partie principale de couche (50) sans fin dans une première direction longitudinale (18) jusqu'à une station d'application (24),
- amener une bande de partie latérale de couche (22) sans fin comprenant une première matière support (30) sans fin, dans laquelle bande il est prévu de façon alternée par rapport à la première matière support (30) des zones extensibles (70) dans le sens longitudinal de la bande de partie latérale de couche (22), c'est-à-dire à distance les unes des autres,
- séparer des segments longitudinaux (46) de la bande de partie latérale de couche (22) et amener les segments longitudinaux (46) jusqu'à la station d'application (24) et les positionner sur la bande de partie principale de couche (50),
- fixer de manière inamovible les segments longitudinaux (46) de la bande de partie latérale de couche (22) à des premières zones (56) de la bande de partie principale de couche, les premières zones (56) comprenant chacune une zone de hanche avant (12) et une zone de hanche arrière (14) de deux parties principales de couche (4) se suivant dans le sens longitudinal et se délimitant l'une de l'autre,
- séparer les couches jetables pour personnes incontinentes (2) en séparant la bande de partie principale de couche (50) transversalement au sens longitudinal (18), la séparation traversant les segments longitudinaux (46) de telle sorte qu'un premier segment partiel (62) d'un segment longitudinal (46) forme une partie latérale de couche avant (8) d'une première couche jetable pour personnes incontinentes, et qu'un deuxième segment partiel (64) du segment longitudinal correspondant (46) forme une partie latérale de couche arrière (10) d'une deuxième couche jetable pour personnes incontinentes directement adjacente,
- la partie latérale de couche arrière (10) présentant une extensibilité supérieure à la partie latérale de couche avant (8).

2. Procédé selon la revendication 1, **caractérisé en ce que** chaque partie principale de couche (4) présente un noyau d'absorption (6) accumulant les fluides.

3. Procédé selon la revendication 1 ou 2, **caractérisé en ce que** les zones extensibles (70) sont des zones extensibles élastiquement.

4. Procédé selon l'une des revendications précédentes, **caractérisé en ce que** dans un deuxième segment partiel (64) de la bande de partie latérale de couche (22) il est prévu dans le sens longitudinal (18) au moins deux zones extensibles (70a, 70b) à distance l'une de l'autre.

5. Procédé selon l'une des revendications précédentes, **caractérisé en ce qu'**il est joint à la bande de partie principale de couche (50) des premiers éléments élastiques (20) s'étendant dans le premier sens longitudinal (18).

6. Procédé selon l'une des revendications précédentes, **caractérisé en ce qu'**il est joint à la bande de partie principale de couche (50) des deuxièmes éléments élastiques s'étendant dans le premier sens longitudinal (18).

7. Procédé selon l'une des revendications précédentes, **caractérisé en ce que** les deuxièmes éléments élastiques sont constitués d'éléments à boucles et crochets.

8. Procédé selon l'une des revendications précédentes, **caractérisé en ce que** l'amenée de la bande de partie latérale de couche (22) jusqu'à la station d'application (24) se fait à une première vitesse v1 et **en ce que** l'amenée de la bande de partie principale de couche (50) sans fin jusqu'à la station d'application (24) se fait à une deuxième vitesse v2, la première vitesse v1 étant inférieure à la deuxième vitesse v2.

9. Procédé selon la revendication 8, **caractérisé en ce que** la deuxième vitesse v2 est supérieure d'au moins 40 %, en particulier d'au moins 70 %, en particulier d'au moins 90 %, et en particulier d'au maximum 200 % à la première vitesse v1.

10. Procédé selon l'une des revendications précédentes, **caractérisé en ce que** les zones élastiques (70) de la bande de partie latérale de couche (22) sont formées par application d'une matière élastique (36) sur la matière support (30) et par étirement par endroits de la matière support (30).

11. Procédé selon l'une des revendications précédentes, **caractérisé en ce que** la matière élastique de la bande de partie latérale de couche (22) est formée par une matière élastique (36) s'étendant de façon continue dans le sens longitudinal de la bande de partie latérale de couche et en particulier en formant des stries, mais **en ce qu'**elle n'est activée que par endroits dans le sens longitudinal.

12. Procédé selon l'une des revendications précédentes, **caractérisé en ce que** la matière élastique (36) est agencée en sandwich entre une première et une deuxième matière support.

13. Procédé selon l'une des revendications précédentes, **caractérisé en ce qu'**un segment longitudinal (46) de la bande de partie latérale de couche (22) est réalisé continu d'un seul tenant dans le sens transversal de la couche (2), de sorte qu'il couvre en continu la zone de hanche avant ou arrière concernée (12, 14) de la partie principale de couche (4).

14. Procédé selon l'une des revendications précédentes, **caractérisé en ce que**, pour la bande de partie latérale de couche (22), il est prévu successivement dans le sens longitudinal un segment longitudinal (46a) pour former une partie latérale de couche droite et un segment longitudinal (46b) pour former une partie latérale de couche gauche.

15. Procédé selon l'une des revendications précédentes, **caractérisé en ce que** lors de la découpe des couches jetables pour personnes incontinentes (2) ou lors de la séparation des segments longitudinaux (46), il se forme un segment à jeter (72, 80).

16. Procédé selon l'une des revendications précédentes, **caractérisé en ce que** lors de la découpe on réalise une coupe curviligne (78).

17. Procédé selon l'une des revendications précédentes, **caractérisé en ce que** la partie latérale de couche arrière présente, avec une application de force de 45 N, un étirement d'au moins 20 %, en particulier d'au moins 25 %, et plus particulièrement d'au moins 30 %.

18. Procédé selon l'une des revendications précédentes, **caractérisé en ce que** la partie latérale de couche avant, avec une application de force de 45 N, présente un étirement au maximum de 15 %, en particulier au maximum de 10 %, et plus particulièrement au maximum de 8 %.

19. Procédé selon l'une des revendications précédentes, **caractérisé en ce que** les parties latérales de couche avant (8) sont réalisées essentiellement non extensibles.

20. Procédé selon l'une des revendications précédentes, **caractérisé en ce que** les parties latérales de couche (8, 10) sont pliées autour d'au moins une, en particulier autour de deux lignes de pliage s'étendant dans le premier sens longitudinal (18).

21. Procédé selon l'une des revendications précédentes, **caractérisé en ce que** les parties latérales de couche (8, 10) sont pliées sur elles-mêmes et fixées de façon amovible dans cette configuration.

22. Procédé selon l'une des revendications précédentes, **caractérisé en ce que** les couches jetables pour personnes incontinentes (2) découpées sont pliées autour d'au moins une, en particulier autour de deux lignes de pliages s'étendant transversalement au premier sens longitudinal (18).
